# EUROPÄISCHE PATENTSCHRIFT

(11) **EP 2 531 122 B1**
(45) Veröffentlichungstag und Bekanntmachung des Hinweises auf die Patenterteilung: **19.03.2014**
(21) Anmeldenummer: 11715387.4
(22) Anmeldetag: 29.01.2011
(51) Int. Cl.: A61B 17/34, A61B 10/04, A61B 19/00, A61B 10/02

(54) **VORRICHTUNG ZUM FESTLEGEN DER EINDRINGTIEFE EINES ROHR- ODER STANGENFÖRMIGEN SCHIEBETEILES IN EINEM AUFNAHMETEIL UND SOLCHE VORRICHTUNGEN VERWENDENDES MEDIZINISCHES HANDSTÜCK**
DEVICE FOR ESTABLISHING THE PENETRATION DEPTH OF A TUBULAR OR ROD-SHAPED SLIDING PART IN A RECEIVING PART AND MEDICAL HANDPIECE USING SUCH DEVICES
DISPOSITIF DE DÉTERMINATION DE LA PROFONDEUR DE PÉNÉTRATION D'UN ÉLÉMENT COULISSANT EN FORME DE TUBE OU DE TIGE DANS UN ÉLÉMENT RÉCEPTEUR ET PIÈCE À MAIN MÉDICALE COMPORTANT DE TELS DISPOSITIFS

(30) Priorität: 02.02.2010 DE 102010006627; 19.07.2010 DE 102010027578; 14.08.2010 DE 102010034408
(43) Veröffentlichungstag der Anmeldung: 12.12.2012
(73) Patentinhaber: Medi-Globe GmbH, 83101 Rohrdorf-Achenmühle (DE)
(72) Erfinder: FINK, Peter Paul, 83737 Irschenberg (DE)
(74) Vertreter: Nätebusch, Roderich
(86) Internationale Anmeldenummer: PCT/DE2011/000090
(87) Internationale Veröffentlichungsnummer: WO 2011/095156

(56) Entgegenhaltungen:
- EP-A2- 1 849 415
- EP-A2- 2 030 574

## Beschreibung

Die vorliegende Erfindung bezieht sich auf eine Vorrichtung zum Festlegen der Eindringtiefe eines rohr- oder stangenförmigen Schiebeteiles, insbesondere eines medizinischen Aspirationsnadel-Trägerteiles, in einem Aufnahmeteil mittels eines das Schiebeteil umgebenden Einstellelements, welches in Längsrichtung des Schiebeteiles verstellbar und in seiner jeweiligen Stellung mittels einer Klemmeinrichtung unter Festlegung der Eindringtiefe des Schiebeteiles in dem Aufnahmeteil blockierbar ist. Ferner bezieht sich die vorliegende Erfindung auf ein medizinisches Handstück, in welchem eine erste und eine zweite Vorrichtung der vorstehend genannten Art verwendet sind.

Eine Vorrichtung der vorstehend genannten Art ist bereits bekannt (JP 01-113070). Bei dieser bekannten Vorrichtung sind dem mit einer Aspirationsnadel verbundenen Schiebeteil und dem Einstellelement im Prinzip in Längsrichtung der Vorrichtung verlaufende, einander gegenüberliegende Rastelemente zugeordnet, die sich zunächst nicht miteinander in Eingriff befinden, wenn sich ein als Klemmeinrichtung dienender, in der Längsrichtung der Vorrichtung verschiebbarer Klemmring in einer ersten Position befindet. In dieser ersten Position ist eine relative Verschiebung des Schiebeteiles und damit der Aspirationsnadel relativ zu einem diese aufnehmenden Aufnahmeteil, welches mit einem Lumen eines Endoskops verbindbar ist, möglich. Sind das Schiebeteil und damit die Aspirationsnadel in eine gewünschte Position längs des Aufnahmeteiles geschoben, so kann der Klemmring in Längsrichtung der Vorrichtung in eine von der genannten ersten Position verschiedene zweite Position verschoben werden, in der die erwähnten Rastelemente gewissermaßen einrasten, so dass das Schiebeteil und damit die Aspirationsnadel nicht mehr relativ zu dem genannten Aufnahmeteil verschiebbar sind.

Grundsätzlich ist bei der gerade betrachteten bekannten Vorrichtung eine Einhandbedienung möglich, um sowohl eine gewünschte Einstellposition des Schiebeteiles und damit der Aspirationsnadel relativ zu dem diese aufnehmenden Aufnahmeteil als auch eine Arretierung und damit Blockierung des Schiebeteiles in einer gewünschten Einstellposition in Bezug auf das Aufnahmeteil zu erreichen. Allerdings sind für die Ausführung beider Funktionen Verschiebebewegungen jeweils in Längsrichtung der Vorrichtung auszuführen. Dadurch kann es bei der Verschiebung des Klemmringes - bei in der gewünschten Einstellposition befindlichem Schiebeteil und damit der Aspirationsnadel - relativ zu dem Aufnahmeteil zu einer ungewollten und damit unerwünschten Verschiebung des Schiebeteiles kommen.

Außerdem wird der für die Realisierung der erläuterten beiden Funktionen (Längsverschiebung des Schiebeteiles in Bezug auf das Aufnahmeteil und Arretierung des Schiebeteiles mittels des Klemmringes) erforderliche apparative Aufwand zuweilen als zu hoch angesehen. Es besteht daher vielfach der Wunsch nach einer Vorrichtung mit einem gegenüber dem Aufbau der betreffenden bekannten Vorrichtung vereinfachten Aufbau.

Es ist ferner bereits ein Handstück für ein medizinisches Gerät bekannt (US 6.976.955 B2, WO 2005/004730 A1), bestehend aus einem zwei Skaleneinteilungen tragenden inneren Handstückteil mit proximalen und distalen Endteilen, die durch einen auch die beiden Skaleneinteilungen voneinander trennenden Begrenzungsanschlag getrennt sind, einem ersten äußeren Handstückteil, welches auf dem proximalen Endteil des inneren Handstückteiles proximal von dem Begrenzungsanschlag verschiebbar angeordnet ist, einem zweiten äußeren Handstückteil, welches auf dem distalen Endteil des inneren Handstückteiles distal von dem Begrenzungsanschlag verschiebbar angeordnet ist, einer länglichen Umhüllung, die an dem inneren Handstückteil angebracht ist und die sich axial über das distale Ende hinaus erstreckt und die ein Umhüllungslumen festlegt, und einer feinen Sonde, die an dem ersten äußeren Handstückteil angebracht und in dem Umhüllungslumen verschiebbar aufgenommen ist.

Um bei diesem bekannten Handstück die beiden äußeren Handstückteile in Bezug auf das innere Handstückteil in gewünschte Positionen relativ zueinander einzustellen und damit die Position des distalen Endes der feinen Sonde in Bezug auf das distale Ende der diese enthaltenden Umhüllung festzulegen, kann von den diese Tätigkeit ausführenden Personen zuweilen das die beiden erwähnten Skaleneinteilungen tragende innere Handstückteil mit einer Hand erfasst werden, wenn nicht zuvor die Position eines der äußeren Handstückteile durch eine Klemmschraube fixiert ist. Dadurch ist aber wenigstens ein Teilbereich der Skaleneinteilungen abgedeckt, was eine genaue Positionierung der äußeren Handstückteile in Bezug auf das genannte innere Handstückteil erschwert.

Außerdem kann es bei dem betreffenden bekannten Handstück infolge der Anordnung der äußeren Handstückteile an gegenüber liegenden Endbereichen des inneren Handstückteiles zu Orientierungsproblemen dahingehend kommen, welche Wirkungen mit dem Verschieben des jeweiligen äußeren Handstückteiles in Bezug auf den genannten Begrenzungsanschlag auf die feine Sonde und die diese aufnehmende Umhüllung verbunden sind. Im Falle der feinen Sonde führt eine Verschiebung des mit dieser verbundenen äußeren Handstückteiles zu einem Ausfahren ihres distalen Endes, wenn dieses äußere Handstückteil in Richtung zu dem Begrenzungsanschlag auf dem inneren Handstückteil verschoben wird. Zum anderen führt eine Verschiebung des mit der Umhüllung verbundenen anderen äußeren Handstückteiles in Richtung von dem Begrenzungsanschlag weg zu einem Ausfahren des distalen Endes der Umhüllung. In Bezug auf den betreffenden Begrenzungsanschlag des inneren Handstückteiles sind also zwei gegenläufige Bewegungen der beiden äußeren Handstückteile auszuführen, um die distalen Enden der feinen Sonde und der diese aufnehmenden Umhüllung in derselben Richtung zu verschieben.

Es ist schließlich auch schon eine weitere Vorrichtung der eingangs genannten Art bekannt auf welcher der Oberbegriff von Anspruch 1 basiert (EP 2 030 574 A2), die an ihrem distalen Ende eine von einem Hohlrohr aufgenommene, in axialer Hohlrohrrichtung verschiebbare Punktionsnadel aufweist. Auf dem Hohlrohr ist eine in dessen axialer Richtung verstellbare Nadeleinstelleinrichtung vorgesehen, die ein Einstellelement mit einem spitz zulaufenden Eingreifglied aufweist, welches in den Bereich zwischen jeweils zwei Zähnen eines eine Vielzahl von Zähnen aufweisenden Zahnstangenelements eingreift, das sich längs des Hohlrohres auf dessen Außenumfang befindet. Durch Positionieren des Eingreifgliedes längs des Zahnstangenelements ist die jeweilige Eindringtiefe des Hohlrohres in ein am proximalen Vorrichtungsende vorgesehenes Aufnahmerohr festlegbar, welches die Punktionsnadel trägt. Dadurch ist dann die jeweilige Ausfahrlänge der Punktionsnadel aus dem genannten Hohlrohr festlegbar. Das Einstellelement lässt sich dabei aber aus seiner jeweiligen Stellung durch eine relativ geringe Schiebekraft verschieben. Diese Schiebekraft entspricht nämlich im Wesentlichen nur der Anhebekraft, die zum Überwinden des jeweiligen Zahnes des Zahnstangenelements durch das Eingreifglied auszuüben ist. Dadurch lässt sich aber ein ungewolltes Verschieben des Einstellelements nicht ausschließen.

Der Erfindung liegt daher die Aufgabe zu Grunde, einen Weg aufzuzeigen, wie eine Vorrichtung der eingangs genannten Art und ein solche Vorrichtungen enthaltendes Handstück auf einfachere Weise als die eingangs betrachtete bekannte Vorrichtung zu realisieren ist und wie dabei zugleich ein ungewolltes Verschieben des Klemmelements nach erfolgter Positionierung des Schiebeteiles relativ zu dem Aufnahmeteil zumindest weitgehend zu verhindern ist. Außerdem soll ein Weg aufgezeigt werden, wie die betreffende Vorrichtung zu einem Handstück für ein medizinisches Gerät ohne die oben im Zusammenhang mit dem bekannten Handstück aufgezeigten Probleme und somit mit einer gegenüber dem betreffenden bekannten Handstück besseren Handhabbarkeit genutzt bzw. erweitert werden kann. Es ist also auf der Grundlage der durch die Erfindung zu schaffenden Vorrichtung ein solches Handstück für ein medizinisches Gerät zu realisieren, das vor allem ohne Orientierungsprobleme für Bedienpersonen sicher zu bedienen ist.

Gelöst wird die vorstehend angegebene Aufgabe bei einer Vorrichtung der eingangs genannten Art gemäß der Erfindung dadurch, dass die Klemmeinrichtung dadurch gebildet ist, dass zwischen dem Einstellelement und dem Schiebeteil zumindest ein Wippenelement angeordnet ist, welches durch Verdrehen des Einstellelements quer zur Längsrichtung des Schiebeteiles zwischen einer die Verschiebbarkeit des Einstellelements in Längsrichtung des Schiebeteiles zulassenden ersten Drehposition und einer eine solche Verschiebbarkeit blockierenden zweiten Drehposition bewegbar ist, die von der ersten Drehposition verschieden ist.

Die Erfindung bringt den Vorteil mit sich, dass bei der durch sie geschaffenen Vorrichtung mit einem einfacheren Konstruktionsprinzip ausgekommen werden kann als es bei der eingangs betrachteten bekannten Vorrichtung angewandt ist. Im Unterschied zu dem bei der betreffenden bekannten Vorrichtung angewandten Konstruktionsprinzip kommt die vorliegende Erfindung mit zumindest einem Wippenelement aus, welches durch Verdrehen des Einstellelements quer zur Längsrichtung des Schiebeteiles zwischen der die Verschiebbarkeit des Einstellelements in Längsrichtung des Schiebeteiles zulassenden ersten Drehposition und der eine solche Verschiebbarkeit blockierenden zweiten Drehposition bewegbar ist, die von der ersten Drehposition verschieden ist.

Die Blockierung des Einstellelements in seiner jeweils gewünschten Einstellposition kann dabei im einfachsten Fall durch Ausnutzung von Reibung zwischen zumindest einer Anlagefläche der von dem Einstellelement umschlossenen jeweiligen Wippenelement und einer dieser Anlagefläche gegenüber liegenden Anlagefläche des Schiebeteiles erreicht werden. Es sind jedoch, wie dies weiter unten noch näher ersichtlich werden wird, auch andere Möglichkeiten vorhanden, um die erwähnte Blockierung des Einstellelements in seiner jeweils gewünschten Einstellposition sogar noch wirksamer sicherzustellen.

Die Erfindung bringt überdies den Vorteil mit sich, dass durch die kombinierte Anwendung der Längsverschiebung des Schiebeteiles in Bezug auf das Aufnahmeteil und der Drehbewegung des Klemmelements die Gefahr eines ungewollten Verschiebens des Klemmelements nach erfolgter Positionierung des Schiebeteiles relativ zu dem Aufnahmeteil zumindest weitgehend verhindert ist.

Gemäß zweckmäßiger Weiterbildung der Erfindung ist für das Blockieren der Verschiebbarkeit des Einstellelements längs des Schiebeteiles eine Zahneinrichtung an bzw. in dem Wippenelement oder an bzw. in dem Schiebeteil vorgesehen, wobei diese Zahneinrichtung in der die Verschiebbarkeit des Einstellelements blockierenden zweiten Drehposition des Einstellelements an dem ihr gegenüber liegenden Bereich des Schiebeteiles bzw. des Wippenelements anliegt oder in diesen Bereich eindringt. Hierdurch ergibt sich der Vorteil einer relativ einfachen und vielfach bereits ausreichend sicheren Blockiermöglichkeit der Verschiebbarkeit des Einstellelements längs des Schiebeteiles.

Vorzugsweise ist das zuvor genannte Einstellelement durch einen Einstellring gebildet, der in seinem das Wippenelement aufnehmenden Innenbereich derart ovaloder sichelförmig geformt ist, dass die an dem Wippenelement oder an dem Schiebeteil vorgesehene Zahneinrichtung lediglich in der genannten zweiten Drehposition an dem ihr gegenüber liegenden Bereich des Schiebeteiles bzw. des Wippenelements anliegt oder in diesen Bereich eindringt. Diese Maßnahme bringt den Vorteil mit sich, dass ein relativ einfacher Blockiermechanismus für ein sicheres Blockieren der Verschiebbarkeit des Einstellelements längs des Schiebeteiles erzielbar ist.

Gemäß einer anderen zweckmäßigen Weiterbildung der Erfindung sind für das Blockieren der Verschiebbarkeit des Einstellelements längs des Schiebeteiles sowohl an dem Wippenelement als auch an dem Schiebeteil zueinander passende Zahneinrichtungen vorgesehen, die lediglich bei Eingriff ineinander in der genannten zweiten Drehposition des Einstellelements die Verschiebbarkeit des Einstellelements blockieren. Hierdurch ergibt sich der Vorteil einer besonders sicheren Blockiermöglichkeit der Verschiebbarkeit des Einstellelements längs des Schiebeteiles.

Vorzugsweise ist das zuvor genannte Einstellelement durch einen Einstellring gebildet, der in seinem das Wippenelement aufnehmenden Innenbereich derart ovaloder sichelförmig geformt ist, dass die an dem Wippenelement und dem Schiebeteil zueinander passenden Zahneinrichtungen lediglich in der genannten zweiten Drehposition miteinander in Eingriff gebracht sind und die Verschiebbarkeit des Einstellelements blockieren. Diese Maßnahme trägt zur zusätzlichen Steigerung des Vorteils bei, dass hierdurch ein relativ einfacher Blockiermechanismus für ein sicheres Blockieren der Verschiebbarkeit des Einstellelements längs des Schiebeteiles erzielbar ist.

Vorzugsweise weist der Einstellring auf seiner Innenseite wenigstens einen abstehenden Rippenteil auf, der durch Anlage an dem Zahnsegmentteil den Drehwinkel des Einstellringes auf dem Schiebeteil zu begrenzen gestattet. Dadurch ergibt sich der Vorteil einer relativ einfachen Führung des Einstellringes auf dem Schiebeteil.

Zweckmäßigerweise erstreckt sich der Drehwinkel bei zwei zumindest angenähert einander gegenüberliegenden Rippenteilen auf einen Wert von etwa 180° abzüglich der Winkelbreite, über die sich das oder das jeweilige Wippenelement erstreckt. Hierdurch ergibt sich der Vorteil einer sehr sicheren Führung des Einstellringes auf dem Schiebeteil.

Gemäß weiterer zweckmäßiger Weiterbildung der Erfindung trägt der Einstellring auf seiner Außenseite zumindest einen, vorzugsweise drei Betätigungsnocken. Hierdurch ergibt sich der Vorteil einer sehr einfachen Bedienbarkeit des Einstellringes auf dem Schiebeteil.

Zweckmäßigerweise steht der jeweilige Betätigungsnocken von dem Einstellring radial ab. Dies bringt den Vorteil einer besonders einfachen Bedienbarkeit des Einstellringes auf dem Schiebeteil mit sich.

Gemäß weiterer zweckmäßiger Weiterbildung der Erfindung ist das jeweilige Wippenelement in Längsrichtung des Schiebeteiles verdrehungssicher geführt. Verdrehungssicher bedeutet hier eine Führung in Bezug auf das Schiebeteil. Hierdurch ergibt sich der Vorteil einer stabilen und lagesicheren Verschiebbarkeit des jeweiligen Wippenelements in Längsrichtung des Aufnahmeteiles und damit auch einer Sicherung der zuvor betrachteten Einstell- und Verschiebefunktionen des Schiebeteiles in Bezug auf das Aufnahmeteil.

Gemäß noch weiterer, besonders zweckmäßiger Weiterbildung der Erfindung ist deren Vorrichtung zur Bildung eines medizinischen Handstücks für ein medizinisches Gerät zweifach vorgesehen, nämlich als eine erste Vorrichtung und als eine zweite Vorrichtung, umfassend folgende Merkmale:
- die erste Vorrichtung gestattet die Eindringtiefe eines ersten rohrförmigen Schiebeteiles in einem ersten Aufnahmeteil festzulegen und die zweite Vorrichtung gestattet die Eindringtiefe eines zweiten rohrförmigen Schiebeteiles in einem zweiten Aufnahmeteil festzulegen;
- die erste Vorrichtung und die zweite Vorrichtung sind derart hintereinander angeordnet, dass das erste Schiebeteil der ersten Vorrichtung zumindest zum Teil als zweites Aufnahmeteil für das zweite Schiebeteil der zweiten Vorrichtung ausgebildet ist;

- das erste Schiebeteil und das zweite Schiebeteil sind jeweils von einem Einstelielement umgeben, relativ zu dem das jeweils zugehörige Schiebeteil verschiebbar ist;
- das erste Einstellelement und das zweite Einstellelement sind in ihrer jeweiligen Stellung mittels einer eigenen Klemmeinrichtung unter Festlegung der Eindringtiefe des zugehörigen ersten bzw. zweiten Schiebeteiles in dem dieses aufnehmenden ersten bzw. zweiten Aufnahmeteil blockierbar;
- jede Klemmeinrichtung ist dadurch gebildet, dass zwischen dem jeweiligen Einstellelement und dessen zugehörigen Schiebeteil zumindest ein Wippenelement angeordnet ist, welches durch Verdrehen des betreffenden Einstellelements quer zur Längsrichtung des zugehörigen Schiebeteiles zwischen einer die Verschiebbarkeit des Schiebeteiles in Längsrichtung relativ zu dem zugehörigen Einstellelement zulassenden ersten Drehposition und einer eine solche Verschiebbarkeit blockierenden zweiten Drehposition bewegbar ist, die von der ersten Drehposition verschieden ist;
- mit dem ersten Aufnahmeteil und dem zweiten Schiebeteil sind Elemente des medizinischen Gerätes verbunden.

Hierdurch wird der Vorteil erzielt, dass durch die kombinierte Anwendung von zwei Vorrichtungen gemäß der Erfindung ein einfach und sicher zu handhabendes Handstück für ein medizinisches Gerät bereitgestellt werden kann, bei dem im Zuge der Einstellvorgänge keine Irritationen für das Handhabungspersonal auftreten.

Gemäß einer zweckmäßigen Weiterbildung der Erfindung ist das medizinische Gerät dadurch gebildet, dass mit dem ersten Aufnahmeteil eine medizinische Hohloder Aspirationsnadel verbunden ist, die sich in Längsrichtung durch das erste Aufnahmeteil, das erste Schiebeteil mit dem daran ausgebildeten zweiten Aufnahmeteil und das zweite Schiebeteil hindurch erstreckt, und dass mit dem zweiten Schiebeteil eine die medizinische Hohl- oder Aspirationsnadel umgebende Umhüllung verbunden ist. Auf diese Weise kann in vorteilhafter Weise ein aus einer medizinischen Hohl- oder Aspirationsnadel und einer diese verschiebbar umgebenden Umhüllung bestehendes medizinisches Gerät bereitgestellt werden, wie es insbesondere für endoskopische Arbeiten in Individuen benötigt wird.

Vorzugsweise ist am proximalen Endbereich des ersten Aufnahmeteiles ein Abschluss-Verbindungselement, insbesondere ein Schraubverbindungselement zur Verbindung mit der medizinischen Hohl- oder Aspirationsnadel angeordnet. Dadurch kann die Hohl- oder Aspirationsnadel in vorteilhafter Weise auswechselbar an dem Handstück angebracht werden, was dann besonders wichtig ist, wenn bei einem Arbeitsvorgang unterschiedliche Hohl- oder Aspirationsnadeln zum Einsatz kommen.

Von besonderem Vorteil ist es gemäß weiterer zweckmäßiger Ausgestaltung der Erfindung, dass das erste Schiebeteil und das zweite Schiebeteil jeweils eine oder mehrere Markierungen oder Skaleneinteilungen enthalten. Dadurch lassen sich auf besonders einfache Weise genaue und genau wiederholbare Einstellungen der Schiebeteile in Bezug auf die zugehörigen Einstellelemente vornehmen.

Gemäß einer weiteren zweckmäßigen Ausgestaltung der Erfindung ist am distalen Endbereich des zweiten Schiebeteiles ein Verbindungselement, insbesondere ein Schraubverbindungselement zur Anbringung an einem Gegen-Verbindungselement eines weiteren medizinischen Gerätes, insbesondere eines Arbeitskanals eines Endoskops vorgesehen. Dies bringt den Vorteil mit sich, dass das Handstück auf einfache und sichere Weise zum Arbeiten mit weiteren medizinischen Geräten und insbesondere mit Arbeitskanälen von Endoskopen verbunden werden kann.

Vorzugsweise ist am proximalen Endbereich des ersten Aufnahmeteiles ein Anschluss-Verbindungselement, insbesondere ein Schraubverbindungselement, gegebenenfalls zur Anbringung eines weiteren medizinischen Gerätes angeordnet. Auch diese Maßnahme trägt dazu bei, dass das Handstück auf einfache und sichere Weise zum Arbeiten mit weiteren medizinischen Geräten, wie mit einer Spritze oder einer Absaugeinrichtung oder einer Spüleinrichtung verbunden werden kann.

Zweckmäßigerweise ist das erste Aufnahmeteil als Griffteil gestaltet. Dies erleichtert die Handhabbarkeit des Handstücks bei der Einstellung der beiden Schiebeteile.

Von ganz besonderem Vorteil ist es, den als zweites Aufnahmeteil für das zweite Schiebeteil der zweiten Vorrichtung ausgebildeten Teil des ersten Schiebeteiles als Griffteil zu gestalten. Dadurch ergibt sich eine äußerst günstige Handhabbarkeit des Handstücks bei der Einstellung der beiden Schiebeteile; bei diesen Einstellungen erfolgt kein Erfassen der Schiebeteile von Hand durch die Person, welche die betreffenden Einstellungen vornimmt.

An Hand von Zeichnungen wird die Erfindung nachstehend an Ausführungsbeispielen näher erläutert.

In den Zeichnungen zeigen
- Fig. 1: eine schematische Draufsicht auf eine Vorrichtung gemäß einer Ausführungsform der Erfindung in einer Größe, die von der in der Praxis verwendeten Größe verschieden sein kann,
- Fig. 2: eine in Bezug auf die Darstellung in Fig. 1 vergrößerte Seitenansicht der betreffenden Vorrichtung entsprechend einer in Fig. 1 eingetragenen Schnittebene II - II, wobei die Vorrichtung mit einem in einer ersten Drehposition befindlichen Einstellelement veranschaulicht ist,
- Fig. 3: eine in Bezug auf die Darstellung in Fig. 1 vergrößerte Seitenansicht der betreffenden Vorrichtung entsprechend der in Fig. 1 eingetragenen Schnittebene II - II, wobei die Vorrichtung mit dem in einer zweiten Drehposition befindlichen Einstellelement veranschaulicht ist,
- Fig. 4: einen Ausschnitt in perspektivischer Darstellung einer Vorrichtung gemäß einer weiteren Ausführungsform der Erfindung bei in einer ersten Drehposition befindlichem Einstellelement dieser Vorrichtung,
- Fig. 5: einen Ausschnitt in perspektivischer Darstellung der Vorrichtung gemäß Fig. 4 bei in einer zweiten Drehposition befindlichem Einstellelement dieser Vorrichtung,
- Fig. 6: in nicht maßstäblichem Maßstab eine Perspektivansicht eines zwei Vorrichtungen gemäß der Erfindung enthaltenden Handstücks entsprechend einer Ausführungsform der Erfindung und
- Fig. 7: in nicht maßstäblichem Maßstab eine Explosionsansicht des in Fig. 6 dargestellten Handstücks.

Bevor auf die Zeichnungen näher eingegangen wird, sei angemerkt, dass in sämtlichen Zeichnungen gleiche oder einander entsprechende Einrichtungen bzw. Elemente mit gleichen oder entsprechenden Bezugszeichen bezeichnet sind.

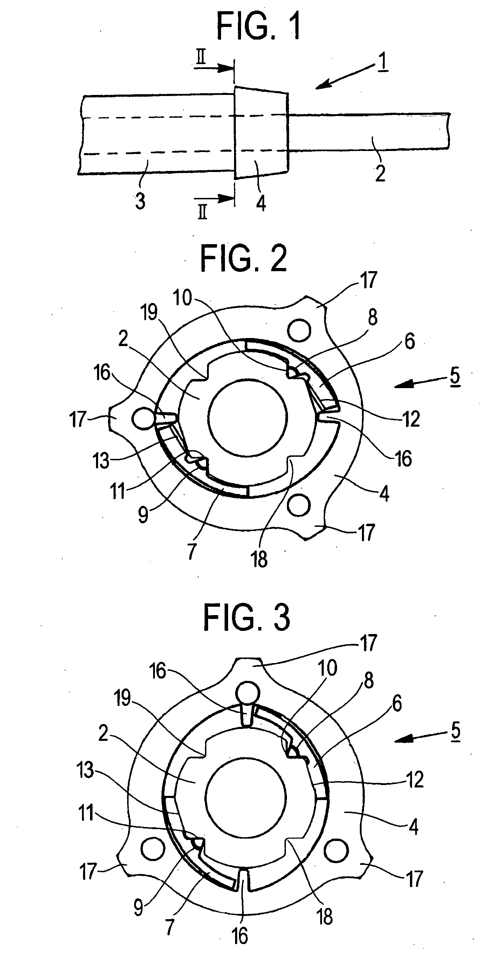

In Fig. 1 ist eine Vorrichtung 1 gemäß einer Ausführungsform der Erfindung in einer schematischen Draufsicht dargestellt. Die Vorrichtung 1 dient zum Festlegen der Eindring- bzw. Eintauchtiefe eines rohr- oder stangenförmigen Schiebeteiles 2 in einem Aufnahmeteil 3 mittels eines das Schiebeteil 2 umgebenden Einstellelements 4. Das Aufnahmeteil 3 weist eine hier nicht näher bezeichnete Öffnung zur verschiebbaren Aufnahme des Schiebeteiles 2 auf; es kann selbst beispielsweise rohrförmig ausgebildet sein. Das Schiebeteil 2 weist im vorliegenden Fall einen kreisrunden Querschnitt auf, der - wie noch ersichtlich werden wird - durch in Längsrichtung des Schiebeteiles 2 geradlinig verlaufende Nuten unterbrochen ist. Das Schiebeteil 2, das Aufnahmeteil 3 und das Einstellelement 4 können jeweils aus Kunststoff bestehen.

Das Einstellelement 4 ist grundsätzlich in Längsrichtung des Schiebeteiles 2 verstellbar und in seiner jeweiligen Stellung mittels einer in Fig. 1 nicht sichtbaren, aber nachstehend an Hand der Fig. 2 und 3 noch näher zu betrachtenden Klemmeinrichtung 5 unter Festlegung der Eindring- bzw. Eintauchtiefe des Schiebeteiles 2 in dem Aufnahmeteil 3 blockierbar.

An dieser Stelle sei noch angemerkt, dass die Vorrichtung 1 in Fig. 1 in einer Größe dargestellt ist, die von einer in der Praxis benutzten Vorrichtung verschieden sein kann. So kann die betreffende Vorrichtung 1 beispielsweise bei der Aufnahme eines das Schiebeteil 2 bildenden medizinischen Aspirationsnadel-Trägerteiles Querabmessungen oder Durchmesser aufweisen, die halb so groß sind wie sie aus Fig. 1 ersichtlich sind. In diesem Fall kann das Aspirationsnadel-Trägerteil an seinem proximalen (in Fig. 1 rechts liegenden) Ende zur Aufnahme der Nadel einer Injektionsspritze dienen, und das das Schiebeteil 2 mit einer an dessen distalen (in Fig. 1 links liegenden) Ende angebrachten Aspirationsnadel aufnehmende Aufnahmeteil 3 kann mit einem Lumen eines (nicht dargestellten) Endoskops verbunden sein, beispielsweise mittels einer Luer-Lock-Verbindung.

In Fig. 2 und 3 ist die zuvor erwähnte Klemmeinrichtung 5 in ihrem Aufbau näher veranschaulicht. Die beiden Fig. 2 und 3 zeigen jeweils in stark vergrößerter Seitenansicht die Vorrichtung 1 entsprechend der Pfeilrichtung der in Fig. 1 eingetragenen Schnittebene II - II, also in dem Bereich des das Schiebeteil 2 umgebenden Einstellelements 4, mit dem dieses Einstellelement 4 an einer Anlagefläche des Aufnahmeteils 3 anzuliegen vermag. Das Einstellelement 4 weist einen Öffnungsbereich auf, der im vorliegenden Fall oval-symmetrisch geformt ist. Der betreffende Öffnungsbereich weist zwei relativ nah beieinander liegende Bereiche und zwei dazwischen relativ weit auseinander liegende Bereiche auf.

Wie aus Fig. 2 und 3 ersichtlich ist, umfasst die Klemmeinrichtung 5 im vorliegenden Fall zwei zwischen dem Einstellelement 4 und dem Schiebeteil 2 angeordnete Wippenelemente 6 und 7, welche durch Verdrehen des Einstellelements 4 quer zu dessen Längsrichtung zwischen einer die Verschiebbarkeit des Einstellelements 4 in Längsrichtung des Schiebeteiles 2 zulassenden ersten Drehposition (Fig.2) und einer eine solche Verschiebbarkeit blockierenden zweiten Drehposition (Fig. 3) bewegbar sind, die von der ersten Drehposition verschieden ist. Es sei hier angemerkt, dass gemäß der Erfindung prinzipiell mit wenigstens einem den Wippenelementen 6 und 7 entsprechenden Wippenelement ausgekommen werden kann. Der Einsatz von zwei oder mehr symmetrisch um das Schiebeteil 2 platzierten Wippenelementen bringt jedoch den Vorteil einer einfacheren und problemlosen Drehbarkeit des Einstellelements 4 mit sich. Das jeweilige Wippenelement kann aus Kunststoff oder einem Metall bestehen.

Die beiden Wippenelemente 6 und 7 weisen, wie aus Fig. 2 und 3 ersichtlich ist, in Längsrichtung der Vorrichtung 1 einen sichelförmigen Querschnitt auf. In ihren mittleren, innen liegenden Bereichen sind die Wippenelemente 6 und 7 mit Lagerzapfen 8 bzw. 9 versehen, die von Lagernuten 10 bzw. 11 im Außenumfang des Schiebeteiles 2 so aufgenommen sind, dass die Wippenelemente 6 und 7 um diese Lagernuten 10 bzw. 11 gekippt werden können. Das jeweilige Wippenelement 6, 7 ist dabei in Längsrichtung des Aufnahmeteiles 3 drehsicher in dem Schiebeteil 2 geführt.

In ihren einen Bereichen, die an den Lagerzapfen 8 bzw. 9 der Wippenelemente 6 und 7 angrenzen, sind die Wippenelemente 6 und 7 mit in Fig. 2 und 3 zur Außenumfangsseite des Schiebeteiles 2 verdickt ausgebildeten Anlagebereichen 12 bzw. 13 versehen. Mit diesen Anlagebereichen 12 und 13 können die Wippenelemente 6 und 7 durch Verdrehen des Einstellelements 4 an dessen Außenumfangsseite in Anlage gebracht werden. Die betreffenden Anlagebereiche 12 und 13 können dabei jeweils mit ihren dem Schiebeteil 2 zugewandten Flächen wie Bremskeile wirken, und zwar entweder mit ihrer normalen Materialoberfläche oder mit einem auf diese Oberfläche aufgebrachten Bremsbelag. Dadurch kann das Schiebeteil 2 in seiner Verschiebbarkeit in das Aufnahmeteil 3 hinein blockiert werden.

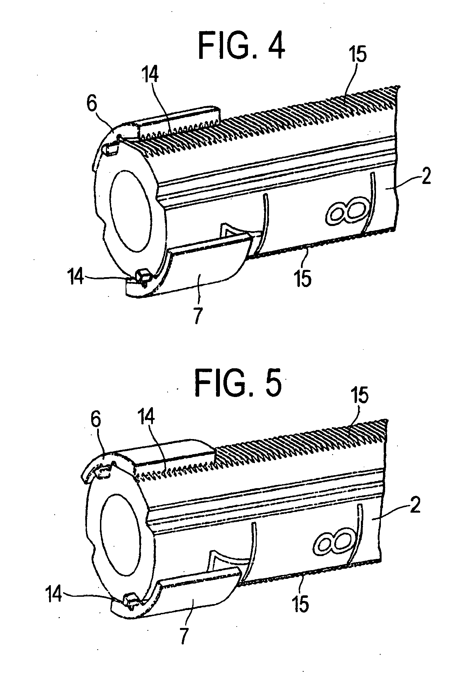

Abweichend von der vorstehend erläuterten Ausbildung und Nutzung der Anlagebereiche 12 und 13 zum Blockieren der Verschiebbarkeit des Schiebeteiles 2 in das Aufnahmeteil 3 hinein (Festlegen der Eindringtiefe) kann alternativ auch so vorgegangen sein, dass an dem Wippenelement 6 bzw. 7 oder an dem Schiebeteil 2 eine Zahneinrichtung 14 oder 15 vorgesehen ist, wie dies in Fig. 4 und 5 veranschaulicht ist. Diese Zahneinrichtung liegt in der die Verschiebbarkeit des Einstellelements 4 blockierenden zweiten Drehposition des Einstellelements 4 an dem ihr gegenüberliegenden Bereich des Schiebeteiles 2 bzw. des Wippenelements 6 bzw. 7 an oder dringt in diesen Bereich ein. In diesem Fall wird die Zahneinrichtung 14 oder 15 vorzugsweise aus einem relativ harten Material, wie aus Metall bestehen, und der Bereich, an dem die betreffende Zahneinrichtung zur Anlage zu bringen ist oder in den die Zahneinrichtung eindringt, wird vorzugsweise aus einem relativ weichen Material, wie aus Kunststoff bestehen.

Ergänzend ist zu der zuvor erwähnten Alternative bezüglich der Darstellung gemäß Fig. 2 und 3 noch anzumerken, dass das Einstellelement 4 dort durch einen Einstellring 4 gebildet ist, der in seinem das Wippenelement 6 bzw. 7 aufnehmenden Innenbereich derart oval- bzw. sichelförmig geformt ist, dass die an dem Wippenelement 6 bzw. 7 oder an dem Schiebeteil 2 vorgesehene Zahneinrichtung 14 bzw. 15 lediglich in der genannten zweiten Drehposition des an dem ihr gegenüberliegenden Bereich des Schiebeteiles bzw. des Wippenelements anliegt oder in diesen Bereich eindringt.

In Weiterbildung der zuletzt betrachteten Ausbildung des jeweiligen Wippenelements 6 bzw. 7 oder des Schiebeteiles 2 zum Blockieren der Verschiebbarkeit des Schiebeteiles 2 in das Aufnahmeteil 3 hinein können sowohl an dem jeweiligen Wippenelement 6, 7 als auch an dem Schiebeteil 2 zueinander passende Zahneinrichtungen 14 bzw. 15 vorgesehen sein, die lediglich bei Eingriff ineinander in der oben erwähnten zweiten Drehposition des Einstellelements 4 dessen Verschiebbarkeit blockieren. Die beiden Zahneinrichtungen 14 bzw. 15 sind zusammen mit den Wippenelementen 6 und 7 und einem Teilabschnitt des Schiebeteiles 2 in Fig. 4 und 5 jeweils in perspektivischer Darstellung veranschaulicht. Das Schiebeteil 2 ist dabei über seine Länge mit einer Skaleneinteilung versehen, die in Verbindung mit dem aus Fig. 4 und 5 weggelassenen Einstellelement bzw. -ring 4 eine genaue und leicht wiederholbare Festlegung der Eindringtiefe des Schiebeteiles 2 in dem Aufnahmeteil 3 ermöglicht. Dabei kann unter Zugrundelegung der Ansicht gemäß Fig. 4 und 5 die rechte Außenseite des die Wippenelemente 6 und 7 umgebenden Einstellelements bzw. -ringes 4 als Messkante für die erwähnte Skaleneinteilung genutzt werden. Die Zahneinrichtung 14 befindet sich dabei jeweils an der Unterseite des verdickten Bereiches der Wippenelemente 6, 7, und sie erstreckt sich hier über deren gesamte Breite. Die Zahneinrichtung 15 erstreckt sich auf der Oberseite und der Unterseite des Schiebeteiles 2 über diejenige Länge, über die dieses Schiebeteil 2 in dem in Fig. 1 dargestellten Aufnahmeteil 3 verschiebbar sein soll, beispielsweise über dessen gesamte Länge. Die Zahneinrichtung 14 kann beispielsweise aus einem harten Kunststoff oder aus Metall bestehen, und die Zahneinrichtung 15 kann aus einem harten Kunststoff bestehen.

Aus Fig. 4 ist dabei der Zustand ersichtlich, in welchem sich die beiden Zahneinrichtungen 14 bzw. 15 nicht miteinander in Eingriff befinden. In diesem Zustand ist das Schiebeteil 2 in dem in Fig. 1 dargestellten Aufnahmeteil 3 leicht verschiebbar. Die Wippenteile 6 und 7 gleiten hierbei unbehindert mit ihren Lagerzapfen 8 bzw. 9 in den Lagernuten 10 bzw. 11 des Schiebeteiles 2 (siehe hierzu die Fig. 2 und 3).

Aus Fig. 5 ist der Zustand ersichtlich, in welchem sich die beiden Zahneinrichtungen 14 bzw. 15 miteinander in Eingriff befinden. In diesem Zustand ist das Schiebeteil 2 in das in Fig. 1 dargestellte Aufnahmeteil 3 nicht mehr hinein oder aus diesem heraus verschiebbar. Die Wippenteile 6 und 7 blockieren eine solche Verschiebbarkeit und legen damit die Eindringtiefe des Schiebeteiles 2 in dem Aufnahmeteil 3 fest.

Ergänzend ist unter Bezugnahme auf die Darstellungen gemäß Fig. 2 und 3 noch anzumerken, dass das Einstellelement 4 durch einen Einstellring 4 gebildet ist, der in seinem das Wippenelement 6 bzw. 7 aufnehmenden Innenbereich derart sichelförmig geformt ist, dass die an dem Wippenelement 6, 7 und dem Schiebeteil 2 zueinander passenden Zahneinrichtungen 14, 15 lediglich in der bereits genannten zweiten Drehposition miteinander in Eingriff gebracht sind und dabei die Verschiebbarkeit des Einstellelements 4 blockieren.

Der erwähnte Einstellring 4 weist auf seiner Innenseite wenigstens einen - im vorliegenden Fall angesichts des symmetrischen Aufbaus der Innenseite des Einstellringes 4 - zwei abstehende Rippenteile 16 auf, die jeweils durch Anlage an dem Wippenteil 6 bzw. 7 den Drehwinkel des Einstellringes 4 auf dem Schiebeteil 2 zu begrenzen gestatten. Dies ist aus einem Vergleich der Darstellungen in den Fig. 2 und 3 ersichtlich. Der betreffende Drehwinkel beträgt bei zwei zumindest angenähert einander gegenüberliegenden Rippenteilen 16 etwa 180° abzüglich der Winkelbreite, über die sich das jeweilige Wippenteil 6 bzw. 7 erstreckt.

Der Einstellring 4 weist auf seiner Außenseite zumindest einen, vorzugsweise jedoch - wie aus Fig. 2 und 3 ersichtlich ist - drei Betätigungsnocken 17 auf, die hier jeweils radial von dem Einstellring 4 abstehen und die eine leichte Bedienbarkeit dieses Einstellringes 4 und damit eine sichere Einhandbedienung der gesamten Vorrichtung 1 hinsichtlich des Festlegens der Eindringtiefe des Schiebeteiles 2 in dem Aufnahmeteil 3 ermöglichen. Diese Einhandbedienung der Vorrichtung 1 ist allerdings grundsätzlich auch ohne die Betätigungsnocken 17 ermöglicht.

Das Schiebeteil 2 weist zusätzlich zu den oben erwähnten Lagernuten 10 bzw. 11 noch zwei weitere jeweils in Längsrichtung des Schiebeteiles 2 verlaufende Nuten 18, 19 auf, die sich allerdings nur über eine begrenzte Länge des Schiebeteiles 2 erstrecken. Mit diesen hier einander diametral gegenüberliegenden und überdies symmetrisch zu den Lagernuten 10 bzw. 11 verlaufenden Nuten 18, 19 wirken Zapfenelemente (nicht dargestellt) zusammen, die an oder in dem Aufnahmeteil 3 enthalten sind. Durch das Eingreifen dieser Zapfenelemente in die Nuten 18, 19 und durch die Verschiebbarkeit des Schiebeteiles 2 in dem Aufnahmeteil 3 lediglich über die erwähnte begrenzte Länge ist sichergestellt, dass das Schiebeteil 2 nicht ungewollt vollständig in das Aufnahmeteil 3 hinein geschoben und aus diesem herausgezogen werden kann: das Schiebeteil 2 ist mit dem Aufnahmeteil 3 gewissermaßen unverlierbar verbunden.

Ferner sei noch angemerkt, dass die Erfindung auf die an Hand der Zeichnungen erläuterten Ausführungsformen nicht beschränkt ist. So können beispielsweise die Funktionen von Einstellring 4 und Schiebeteil 2 entsprechend einer kinematischen Umkehrung vertauscht sein. Dies heißt, dass bei einer solchen Alternative der Einstellring 4 nicht mehr verdrehbar ist und dass sodann das Schiebeteil 2 mit dem jeweiligen Wippenelement 6, 7 zusätzlich zu seiner Längsverschiebbarkeit noch um seine Längsachse drehbar ist. In diesem Fall kann die Innenseite des an Hand der Fig. 2 und 3 betrachteten Einstellringes 4 eine kreisrunde Form besitzen, und das Schiebeteil 2 kann mit einem ovalförmigen Querschnitt versehen sein. Dabei kann es angebracht sein, den Einstellring 4 mit dem Aufnahmeteil 3 fest zu verbinden.

In Abweichung von den vorstehend erläuterten Ausführungsformen kommt die vorliegende Erfindung grundsätzlich mit einem Wippenelement zwischen dem Schiebeteil 2 und dem Einstellelement 4 aus. In diesem Fall braucht die Innenform des Einstellelements 4 nicht oval-symmetrisch geformt zu sein. Vielmehr genügt eine einzige sichelförmige Gestaltung eines Teiles der Öffnung des Einstellelements 4.

Während bei den vorstehend erläuterten Ausführungsformen sowohl für das Schiebeteil 2 als auch für die dieses Schiebeteil 2 aufnehmende Öffnung des Aufnahmeteiles 3 jeweils runde Querschnitte angenommen worden sind, können jedoch auch andere Querschnittsformen vorgesehen sein, wie zum Beispiel viereckige oder generell polygone Querschnittsformen.

Auch die Zahneinrichtungen 14, 15 können anders gestaltet sein als dies aus Fig. 4 und 5 ersichtlich ist. So braucht beispielsweise die Zahneinrichtung 14 sich nicht über die gesamte Breite des jeweiligen Wippenelements 6 bzw. 7 zu erstrecken. Grundsätzlich kann mit nur einem Zahnelement in/an dem jeweiligen Wippenelement 6 bzw. 7 oder dem Schiebeteil 2 und mit einer Mehrzahl von Zahnelement-Aufnahmen in/an dem Schiebeteil 2 oder dem jeweiligen Wippenelement 6 bzw. 7 ausgekommen werden.

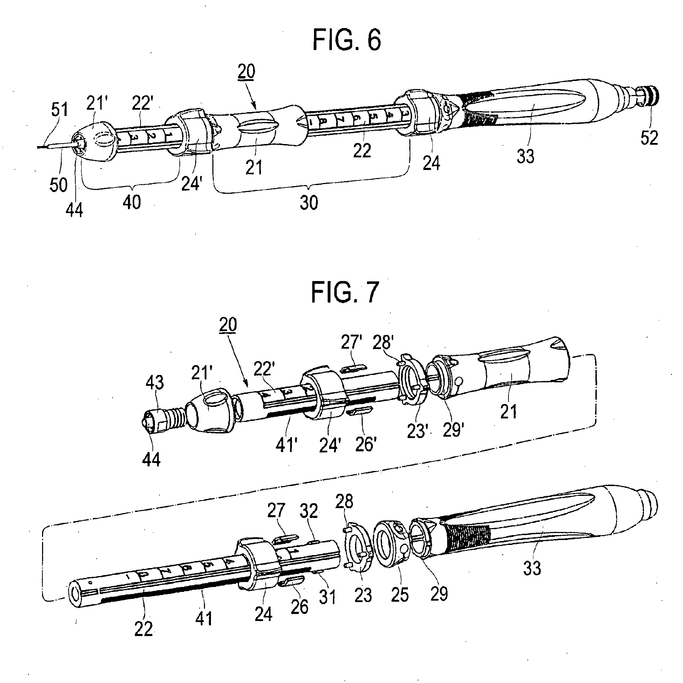

An Hand der Fig. 6 und 7 wird nachstehend erläutert, wie die aus Fig. 1 bis 5 ersichtlichen Konstruktionsprinzipien bei einem medizinischen Handstück 20 für ein medizinisches Gerät zur praktischen Anwendung kommen. Es wird gezeigt, wie Vorrichtungen gemäß der Erfindung in der Praxis bei einem medizinischen Handstück verwendet werden.

In Fig. 6 ist das Handstück 20 gemäß einer Ausführungsform der Erfindung für ein medizinisches Gerät in auseinander gezogenem, arbeitsmäßigen Zustand gezeigt. Bei diesem medizinischen Gerät handelt es sich beim vorliegenden Ausführungsbeispiel um eine medizinische Hohlnadel oder Aspirationsnadel 51, die von einer Umhüllung 50 umgeben und in dieser verschiebbar ist. Die betreffende Umhüllung 50 ist, wie nachstehend noch ersichtlich werden wird, ebenfalls auf der medizinischen Hohlnadel oder Aspirationsnadel 51 verschiebbar; sie kann zumindest an ihrem distalen Ende - das ist das in Fig. 6 links dargestellte Ende - spiralförmig ausgebildet sein. Die betreffende Hohl- oder Aspirationsnadel 51 ist im vorliegenden Fall mit ihrem proximalen Ende mit einem ersten Aufnahmeteil oder proximalen Griffteil 33 verbunden. Ihr proximales Ende ist überdies mit einem proximalen Abschlussteil 52 verbunden, an das beim vorliegenden Ausführungsbeispiel ein weiteres medizinisches Gerät angeschlossen werden kann, wie zum Beispiel eine Spritze oder eine Absaugeinrichtung oder eine Spüleinrichtung. Das betreffende proximale Abschlussteil 52 kann aber gegebenenfalls auch ein Schraubverschlussfeil sein, das beispielsweise auf ein am proximalen Ende des Handstücks vorgesehenes Gegenschraubteil aufgeschraubt sein kann. Bei der vorliegenden Ausführungsform des Handstücks 20 gemäß der Erfindung ist dieses Schraubverschlussteil mit dem proximalen Ende der medizinischen Hohlnadel oder Aspirationsnadel 51 verbunden.

Es sei an dieser Stelle angemerkt, dass das erwähnte medizinische Gerät auch durch andere medizinische Vorrichtungen gebildet sein kann, wie durch eine Fang- bzw. Klemmvorrichtung oder durch eine Schneidvorrichtung, wie eine Zange oder Schere, und zwar jeweils von einer der Umhüllung 50 entsprechenden Umhüllung aufgenommen und relativ zu dieser verschiebbar.

Das Handstück 20 umfasst zwei Vorrichtungen gemäß der Erfindung. Entsprechend einer ersten Betrachtungsweise umfasst die eine dieser beiden Vorrichtungen - und zwar bei Betrachtung des Handstücks 20 von dessen proximalen Ende aus - das ist das in Fig. 6 rechts dargestellte Ende - ein erstes Schiebeteil 30 und das bereits erwähnte erste Aufnahmeteil bzw. proximale Griffteil 33, in welches das erste Schiebeteil 30 einschiebbar und aus diesem herausziehbar ist. Die andere der beiden Vorrichtungen gemäß der Erfindung umfasst bei dieser Betrachtungsweise ein zweites Schiebeteil 40, welches von dem für dieses Schiebeteil 40 als Aufnahmeteil ausgebildeten und wirkenden ersten Schiebeteil 30 aufgenommen und sowohl in dieses einschiebbar als auch aus diesem herausziehbar ist.

Entsprechend einer zweiten Betrachtungsweise umfasst die eine dieser beiden Vorrichtungen - und zwar bei Betrachtung des Handstücks 20 von dessen distalen Ende aus - das ist das in Fig. 6 links dargestellte Ende - das Schiebeteil 40 und das dieses aufnehmende Aufnahmeteil bzw. Griffteil 30, in welches das genannte Schiebeteil 40 einschiebbar und aus diesem herausziehbar ist. Die andere der beiden Vorrichtungen gemäß der Erfindung umfasst bei dieser Betrachtungsweise das als Schiebeteil wirkende Aufnahmeteil bzw. Griffteil 30, welches von dem proximalen Griffteil 33 aufgenommen und sowohl in dieses einschiebbar als auch aus diesem herausziehbar ist.

Die beiden vorstehend erläuterten Betrachtungsweisen haben gezeigt, dass das proximale Griffteil 33, das Schiebeteil 30 und das Schiebeteil 40 jeweils ein teleskopförmiges Gebilde darstellen, bei dem das Schiebeteil 40 von dem Schiebeteil 30 verschiebbar aufgenommen ist und bei dem das Schiebeteil 30 von dem proximalen Griffteil 33 verschiebbar aufgenommen ist.

Nachdem zuvor unter Bezugnahme auf Fig. 6 der Aufbau des Handstücks 20 generell hinsichtlich der dort eingesetzten beiden Vorrichtungen gemäß der Erfindung erläutert worden ist, wird nunmehr auf den näheren Aufbau dieser beiden Vorrichtungen unter Heranziehung der ersten, oben durchgeführten Betrachtungsweisen eingegangen.

Gemäß Fig. 6 besteht das erste Schiebeteil 30 aus einem zweiten Aufnahmeteil bzw. mittleres Griffteil 21 und einem ersten Schieberohr 22. Dieses Schieberohr 22 enthält im vorliegenden Fall eine Skaleneinteilung; sie kann aber gegebenenfalls auch nur eine Markierung oder mehrere Markierungen oder mehrere Skaleneinteilungen aufweisen. Das Schieberohr 22 ist in der aus Fig. 6 ersichtlichen Position mit dem zweiten Aufnahmeteil bzw. mittleres Griffteil 21 fest verbunden, beispielsweise durch eine Klebverbindung.

Auf dem Schieberohr 22 ist gemäß Fig. 6 ein erstes Einstellelement bzw. Drehglied 24 vorgesehen, welches längs des Schieberohres 22 verstellbar und um dieses verdrehbar ist oder welches alternativ an dem proximalen Griffteil 33 drehbar angebracht und um das Schieberohr 22 drehbar ist. Durch Drehen dieses Einstellelements bzw. Drehgliedes 24 zwischen einer ersten Drehposition und einer davon verschiedenen zweiten Drehposition kann die Verschiebbarkeit des Schieberohres 22 und damit des ersten Schiebeteiles 30 in dem proximalen Griffteil 33 freigegeben oder blockiert und damit die Eindringtiefe des Schieberohres 22 in dem proximalen Griffteil 33 festgelegt werden.

Das zweite Schiebeteil 40 besteht aus einem zweiten Schieberohr 22' und einem an dessen distalen Ende angebrachten Kopfteil 21'. Das Schieberohr 22' und das Kopfteil 21' können beispielsweise durch eine Klebverbindung miteinander fest verbunden sein. Das Schieberohr 22' enthält im vorliegenden Fall eine Skaleneinteilung; sie kann aber gegebenenfalls auch nur eine Markierung oder mehrere Markierungen oder mehrere Skaleneinteilungen aufweisen. Das Schieberohr 22' ist zur Festlegung seiner Eindringtiefe in dem zweiten Aufnahmeteil bzw. mittleres Griffteil 21 in dieses einschiebbar und aus diesem herausziehbar.

Zur Festlegung der jeweiligen Eindringtiefe des Schieberohres 22' in dem zweiten Aufnahmeteil bzw. mittleres Griffteil 21 ist auf dem Schieberohr 22 gemäß Fig. 6 ein zweites Einstellelement bzw. Drehglied 24' vorgesehen, welches an dem distalen Endbereich des zweiten Aufnahmeteiles bzw. mittleren Griffteiles 21 drehbar angebracht ist. Durch Drehen dieses Einstellelements bzw. Drehglieds 24' zwischen einer ersten Drehposition und einer davon verschiedenen zweiten Drehposition kann die Verschiebbarkeit des Schieberohres 22' und damit des zweiten Schiebeteiles 40 in dem ersten Schiebeteil 30 freigegeben oder blockiert und damit die Eindringtiefe des Schieberohres 22' in dem zweiten Aufnahmeteil bzw. mittleren Griffteil 21 des ersten Schiebeteiles 30 festgelegt werden.

In Fig. 6 ist in dem am distalen Ende des Handstücks 20 angebrachten Kopfteil 21' ein Schraubteil 44 dargestellt, bei dem es sich insbesondere um ein Schraubverbindungselement zur Anbringung an einem Gegen-Verbindungselement eines medizinischen Gerätes, insbesondere eines Arbeitskanals eines Endoskops handelt. Dieses Schraubverbindungselement kann ein Luer-Lock-Verbindungselement sein, welches mit einem entsprechenden Gegen-Luer-Lock-Verbindungselement verbindbar ist.

Nachdem der Aufbau des Handstücks 20 an Hand der Fig. 6 in seinem auseinander gezogenen Zustand erläutert worden ist, sei nunmehr kurz auf dessen zusammengeschobenen Zustand eingegangen. Im zusammengeschobenen Zustand des Handstücks 20 liegt der distale Endbereich des zweiten Aufnahmeteiles bzw. mittleren Griffteiles 21 an dem proximalen Endbereich des ersten Einstellelements bzw. Drehgliedes 24 an, und der proximale Endbereich des zweiten Einstellelements bzw. Drehgliedes 24' liegt am distalen Endbereich des Kopfteiles 21' an. Das erste Schieberohr 22 ist vollständig in dem ersten Aufnahmeteil 33 des Handstücks 20 eingeschoben, und das zweite Schieberohr 22' ist vollständig in dem zweiten Aufnahmeteil 21 des Handstücks 20 eingeschoben.

Nunmehr wird auf Fig. 7 Bezug genommen, in der das in Fig. 6 dargestellte Handstück in einer Explosionsansicht veranschaulicht ist, um den Zusammenbau der einzelnen Elemente des Handstücks 20 beispielhaft zu erläutern.

Im unteren Teil der Explosionsansicht gemäß Fig. 7 ist das erste Schieberohr 22 dargestellt, auf dem das erste Einstellelement bzw. Drehglied 24 zwischen zwei Drehpositionen drehbar und in Längsrichtung des Schieberohres 24 verschiebbar aufgenommen. Längs des ersten Schieberohres 24 ist in Fig. 7 eine Zahneinrichtung 41 von zwei zumindest angenähert diametral auf der Außenseite des Schieberohres 24 sich erstreckenden Zahneinrichtungen angedeutet. Diese Zahneinrichtungen stellen zahnstangenartige Elemente dar, in deren Zahnungen ein erstes Wippenelement 26 bzw. ein zweites Wippenelement 27 mit jeweils zumindest einem Zahn durch Drehen des Einstellelements bzw. Drehgliedes 24 in Eingriff bringbar bzw. aus einem solchen Eingriff lösbar sind.

Die beiden Wippenelemente 26 und 27 befinden sich nach dem Zusammenbau des Handstücks 20 zwischen dem ersten Schieberohr 22 und einer ovalförmigen Aufnahmeöffnung in dem Einstellelement bzw. Drehglied 24. Durch die Ausbildung des Einstellelements bzw. Drehgliedes 24 in seinem Innern mit der ovalförmigen Aufnahmeöffnung, ist es, wie dies im Zusammenhang mit den Fig. 1 bis 5 augeführt ist, möglich, die beiden Wippenelemente 26 und 27 mit ihren Zähnungen in einer ersten Drehposition des ersten Einstellelements bzw. Drehgliedes 24 aus den erwähnten Zahneinrichtungen, wie der Zahneinrichtung 41, zu lösen, also außer Eingriff zu bringen, und in einer von dieser ersten Drehposition verschiedenen zweiten Drehposition in Eingriff zu bringen.

Zur zentrierten Anbringung des ersten Einstellelements bzw. Drehgliedes 24 auf dem ersten Schieberohr 22 ist an dem betreffenden Einstellelement bzw. Drehglied 24 ein erster Klemm- bzw. Justierring 23 mit mindestens einem, im vorliegenden Fall mit drei von seiner dem Einstellelement bzw. Drehglied 24 zugewandten Seite abstehenden Zapfen angebracht, die von entsprechenden Aufnahmeöffnungen aufnehmbar sind, welche sich in dem ersten Einstellelement bzw. Drehglied 24 befinden. In Fig. 7 ist einer dieser Zapfen mit 28 bezeichnet.

Wie im Zusammenhang mit Fig. 6 erwähnt, bildet das erste Schieberohr 22 zusammen mit dem zweiten Aufnahmeteil bzw. mittleren Griffteil 21 das erste Schiebeteil 30. Dazu ist das erste Schieberohr 22 mit seinem in Fig. 7 links dargestellten distalen Ende an dem Aufnahmeteil bzw. mittleren Griffteil 21 fest angebracht, beispielsweise in einer entsprechenden Aufnahmeöffnung des Aufnahmeteiles bzw. mittleren Griffteiles 21 und/oder durch Ankleben.

Das erste Schiebeteil 30 ist mit seinem Schieberohr 22 in eine das erste Aufnahmeteil bzw. proximale Griffteil 33 durchziehende Längsöffnung einschiebbar und aus dieser herausziebar. Um bei diesem Herausziehen sicherzustellen, dass das Schieberohr 22 nicht gänzlich aus dem Aufnahmeteil bzw. proximale Griffteil 33 herausgezogen wird, ist ein Blockiermechanismus vorgesehen. Dieser Blockiermechanismus umfasst gemäß Fig. 7 einen Aufnahmering 25 zur Aufnahme des Schieberohres 22 und zwei vom Umfang des Schieberohres 22 radial abstehende Begrenzungswulste 31, 32, die gegebenenfalls durch eine von der Außenfläche des Schieberohres 22 abstehende bogenförmige Klammer gebildet sein können. Der Aufnahmering 25 dient zum Einsetzen in ein Aufnahmeelement bzw. -loch 29 am distalen Ende des proximalen Griffteiles 33. Bevor der Aufnahmering 25 in das Aufnahmeelement bzw. -loch 29 eingesetzt und in diesem befestigt wird, beispielsweise durch eine Klebverbindung, muss jedoch erst das Schieberohr 22 mit seinem distalen Ende durch den Aufnahmering 25 geschoben werden. Auf diese Weise sind dann nach dem Zusammenbauen des Handstücks 20 die von dem Schieberohr 22 radial abstehenden Begrenzungswulste 31, 32 in der Aufnahmeöffnung des proximalen Griffteiles 33 untergebracht. Dadurch, dass die Begrenzungswulste 31, 32 als Anschläge an dem Aufnahmering 25 wirken, kann das Schieberohr 22 nicht mehr vollständig aus dem Griffteil 33 herausgezogen werden.

Nachstehend wird der im oberen Teil der in der Explosionsansicht gemäß Fig. 7 dargestellte Teil des Handstücks 20 näher betrachtet. In diesem Teil ist auf der rechten Seite das zu dem ersten Schiebeteil 30 gehörende Aufnahmeteil bzw. mittlere Griffteil 21 dargestellt. Dieses Aufnahmeteil bzw. mittlere Griffteil 21 ist an seinem distalen Ende mit einem Aufnahmeelement 29' versehen, auf dem ein dem ersten Klemm- bzw. Justierring 23 entsprechender zweiter Klemm- bzw. Justierring 23' drehbar aufsetzbar ist, beispielsweise durch eine Rastverbindung. Der Justierring 23' wird seinerseits mit einem dem ersten Einstellelement bzw. Drehglied 24 entsprechenden zweiten Einstellelement bzw. Drehglied 24' fest verbunden, beispielsweise durch eine Klebverbindung. Zur genauen Ausrichtung des Justierringes 23' an dem zweiten Einstellelement bzw. Drehglied 24' dienen von der distalen Seite des Justierringes 23' abstehende Zapfen, deren einer mit 28' bezeichnet ist und die von entsprechenden Aufnahmelöchern in dem Einstellelement bzw. Drehglied 24' aufgenommen werden.

Bevor die zuvor erwähnte Verbindung zwischen dem Einstellelement bzw. Drehglied 24' und dem Justierring 23' hergestellt wird, sind jedoch den ersten und zweiten Wippenelementen 26, 27 entsprechende dritte und vierte Wippenelemente 26', 27' in den Zwischenraum zwischen einem zweiten Schieberohr 22' und dem Innenraum des dieses Rohr umgebenden Einstellelements bzw. Drehgliedes 24' einzusetzen. Die betreffenden Wippenelemente 26', 27' sind wie die bereits betrachteten Wippenelemente 26 und 27 auf ihren dem Schieberohr 22' zugewandten Seiten mit einer jeweils wenigstens ein Zahnelement umfassenden Zahngebilde versehen, welches von einer längs des Schieberohres 22' auf dessen Außenseite sich erstreckenden zahnstangenartigen Zahneinrichtung aufnehmbar ist. In Fig. 7 ist eine der an dem Schieberohr 22' vorgesehenen beiden zahnstangenartigen Zahneinrichtungen mit 41' bezeichnet.

Das erwähnte Zahngebilde der beiden Wippenelemente 26', 27' ist in einer ersten Drehposition des Einstellelements bzw. Drehgliedes 24' mit der zugehörigen zahnstangenartigen Zahneinrichtung des Schieberohres 22' außer Eingriff gebracht. In dieser Drehposition ist das Schieberohr 22' dadurch für eine Längsverschiebung freigegeben, so dass es dann in seiner Längsrichtung in Bezug auf das zweite Aufnahmeteil bzw. mittlere Griffteil 21 verschoben werden kann. In einer von der ersten Drehposition verschiedenen zweiten Drehposition des Einstellelements bzw. Drehgliedes 24' sind die beiden Wippenelemente 26', 27' mit ihren Zahngebilden mit den zugehörigen zahnstangenartigen Zahneinrichtungen des Schieberohres 22' in Eingriff gebracht. In dieser zweiten Drehposition kann dann das Schieberohr 22' nicht mehr in Bezug auf das zweite Aufnahmeteil bzw. mittlere Griffteil 21 verschoben werden; seine Eindringtiefe in dem zweiten Aufnahmeteil bzw. mittlere Griffteil 21 ist damit festgelegt. Durch dieses Verschieben kann die Ausfahrlänge der mit dem Schieberohr 22' verbundenen Umhüllung 50 am distalen Ende des Handstücks 20 relativ zu der Ausfahrlänge des mit dem ersten Aufnahmeteil bzw. proximalen Griffteil 33 verbundenen medizinischen Gerätes, wie der Hohl- oder Aspirationsnadel 51 eingestellt und damit festgelegt werden.

Um das Festklemmen und das Freigeben des Schieberohres 22' zu bewirken, weist das Einstellelement bzw. Drehglied 24' wie das Einstellelement bzw. Drehglied 24 in seinem Innern einen ovalförmigen Querschnitt auf, der in seiner ersten Drehposition die beiden Wippenelemente 26', 27' mit ihren Zahngebilden mit den zugehörigen zahnstangenartigen Zahneinrichtungen des Schieberohres 22' außer Eingriff und in der zweiten Drehposition in Eingriff bringt.

Damit nach dem Zusammenbau des Handstücks 20 ein vollständiges Herausziehen des Schieberohres 22' aus dem Griffteil 21 verhindert ist, ist im proximalen Endbereich des Schieberohres 22' ebenfalls ein Blockiermechanismus vorgesehen. Dieser Blockiermechanismus kann in entsprechender Weise realisiert sein wie dies im Zusammenhang mit dem Schieberohr 22 erläutert worden ist. Es ist aber auch möglich, hier die dritten und vierten Wippenelemente 26' und 27' mit ihren Lagerzapfen in Führungsnuten des Schieberohrs 22' aufnehmen zu lassen, wie dies in den Fig. 1 bis 5 näher veranschaulicht ist. Diese Führungsnuten enden in einem festgelegten Abstand von beispielsweise etwa 10mm vor dem proximalen Ende des Schieberohrs 22' und üben damit auf die Wippenelemente 26' und 27' und folglich auf das gesamte Einstellelement bzw. Drehglied 24' eine Anschlagfunktion aus.

An dieser Stelle sei angemerkt, dass ein dem zuvor erläuterten, die dritten und vierten Wippenelemente 26' und 27' umfassenden Blockiermechanismus entsprechender Blockiermechanismus auch in Verbindung mit den ersten und zweiten Wippenelementen 26 und 27 vorgesehen sein kann.

Mit dem distalen Ende des Schieberohrs 22' ist das Kopfteil 21' zu verbinden, und zwar vorzugsweise fest, beispielsweise durch eine Klebverbindung. In dem Kopfteil 21' ist schließlich noch ein distales Anschlussteil 43 einzusetzen, beispielsweise durch eine Schraub- und/oder Klebeverbindung. In diesem distalen Anschlussteil 43 ist das in Verbindung mit Fig. 6 bereits erwähnte Schraubteil 44 enthalten, mit dem das Handstück 20 an ein entsprechendes Gegenschraubteil angeschraubt werden kann, welches beispielsweise im Eintrittsbereich eines Arbeitskanals eines Endoskops vorgesehen sein kann.

Bei dem vorstehend an Hand eines Ausführungsbeispiels erläuterten Handstück 20 gemäß der vorliegenden Erfindung kommen also zwei Vorrichtungen 1 gemäß der Erfindung in Kombination zur Anwendung, um die Eindringtiefe jeweils eines rohrförmigen Schiebeteiles in einem Aufnahmeteil mittels eines das jeweilige Schiebeteil umgebenden Einstellelements einzustellen. Die eine oder erste Vorrichtung umfasst hier das erste Aufnahmeteil bzw. proximale Griffteil 33 und das erste Schiebeteil 30. Die andere oder zweite Vorrichtung umfasst hier als zweites Aufnahmeteil bzw. mittlere Griffteil 21 des gerade erwähnten ersten Schiebeteiles 30 und das zweite Schieberohr 22' des zweiten Schiebeteiles 40.

Dadurch, dass bei dem erläuterten Handstück 20 ein medizinisches Gerät, wie insbesondere eine Hohl- oder Aspirationsnadel 51 mit dem ersten Aufnahmeteil bzw. proximalen Griffteil 33 verbunden ist, und dass mit dem am distalen Ende des Handstücks 20 vorgesehenen zweiten Schieberohr 22' des zweiten Schiebeteiles 40 eine das betreffende medizinische Gerät, wie die erwähnte Hohl- oder Aspirationsnadel 51 umgebende Umhüllung 50 verbunden ist, können folgende Einstellungen vorgenommen werden:
1. Durch Festlegen der Eindringtiefe des ersten Schiebeteiles 30 in dem ersten Aufnahmeteil bzw. proximalen Griffteil 33 kann zum einen die Ausfahrlänge des vom distalen Ende des Handstücks 20 heraus geführten Teiles des medizinischen Gerätes, wie der erwähnten Hohl- oder Aspirationsnadel 51 festgelegt werden.
2. Durch Festlegen der Eindringtiefe des zweiten Schieberohres 22' des zweiten Schiebeteiles 40 in dem zweiten Aufnahmeteil bzw. mittleren Griffteil 21 des ersten Schiebeteiles 30 kann zum anderen die Ausfahrlänge des vom distalen Ende des Handstücks 20 herausgeführten Teiles der das medizinische Gerät, wie die erwähnte Hohl- oder Aspirationsnadel 51 umgebenden Umhüllung 50 festgelegt werden.

Diese beiden gerade erläuterten Einstellvorgänge sind für die Durchführung von medizinischen Arbeiten erforderlich, wie sie insbesondere mittels Endoskopen in Individuen ausgeführt werden. Dadurch, dass das erste Schiebeteil 30 den zweiten Aufnahmeteil bzw. mittleren Griffteil 21 aufweist, ist das Vorrichtungen gemäß der Erfindung verwendende Handstück 20 beim Einstellen der Ausfahrlänge der Umhüllung 50 besser zu handhaben als das eingangs betrachtete bekannte Handstück für ein medizinisches Gerät; beim Halten des Handstücks 20 zum Einstellen der Eindringtiefe des zweiten Schieberohres 22' in das zweite Aufnahmeteil bzw. mittlere Griffteil 21 wird nämlich kein Markierungs- oder Skalenbereich der beiden Schieberohre 22 und 22' abgedeckt. Dadurch, dass bei dem Handstück 20 gleichartige Verschiebungen der beiden Schieberohre 22 und 22' auch zu gleichartigen Verschiebungen der Aspirationsnadel 51 und deren Umhüllung 50 führen, treten für das Bedienpersonal des betreffenden Handstücks 20 keine Orientierungsprobleme auf.

### Bezugszeichenliste

- 1: Vorrichtung
- 2: Schiebeteil
- 3: Aufnahmeteil
- 4: Einstellelement, Einstellring
- 5: Klemmeinrichtung
- 6: Wippenelement
- 7: Wippenelement
- 8: Lagerzapfen
- 9: Lagerzapfen
- 10: Lagernut
- 11: Lagernut
- 12: Anlagebereich
- 13: Anlagebereich
- 14: Zahneinrichtung
- 15: Zahneinrichtung
- 16: Rippenteile
- 17: Betätigungsnocken
- 18: Nut
- 19: Nut
- 20: Handstück
- 21: zweites Aufnahmeteil bzw. mittleres Griffteil
- 21': Kopfteil
- 22: erstes Schieberohr
- 22': zweites Schieberohr
- 23: erster Klemm- bzw. Justierring
- 23': zweiter Klemm- bzw. Justierring
- 24: erstes Einstellelement bzw. Drehglied
- 24': zweites Einstellelement bzw. Drehglied
- 25: Aufnahmering
- 26: erstes Wippenelement
- 26': zweites Wippenelement
- 27: drittes Wippenelement
- 27': viertes Wippenelement
- 28: Zapfen
- 28': Zapfen
- 29: Aufnahmeelement
- 29': Aufnahmeelement
- 30: erstes Schiebeteil
- 31: Begrenzungswulst
- 32: Begrenzungswulst
- 33: erstes Aufnahmeteil bzw. proximales Griffteil
- 40: zweites Schiebeteil
- 41: Zahneinrichtung
- 41': Zahneinrichtung
- 43: distales Anschlussteil
- 44: Verbindungselement
- 50: Umhüllung
- 51: medizinische Hohl- oder Aspirationsnadel
- 52: proximales Anschlussteil

## Patentansprüche

1. Vorrichtung (1) zum Festlegen der Eindringtiefe eines rohr- oder stangenförmigen Schiebeteiles (2), insbesondere eines medizinischen Aspirationsnadel-Trägerteiles, in einem Aufnahmeteil (3) mittels eines das Schiebeteil (2) umgebenden Eiristellelements (4), welches in Längsrichtung des Schiebeteiles (2) verstellbar und in seiner jeweiligen Stellung mittels einer Klemmeinrichtung (5) unter Festlegung der Eindringtiefe des Schiebeteiles (2) in dem Aufnahmeteil (3) blockierbar ist,
**dadurch gekennzeichnet, dass** die Klemmeinrichtung (5) dadurch gebildet ist, dass zwischen dem Einstellelement (4) und dem Schiebeteil (2) zumindest ein Wippenelement (6; 7) angeordnet ist, welches durch Verdrehen des Einstellelements (4) quer zur Längsrichtung des Schiebeteiles (2) zwischen einer die Verschiebbarkeit des Einstellelements (4) in Längsrichtung des Schiebeteiles (2) zulassenden ersten Drehposition und einer eine solche Verschiebbarkeit blockierenden zweiten Drehposition bewegbar ist, die von der ersten Drehposition verschieden ist.

2. Vorrichtung nach Anspruch 1, **dadurch gekennzeichnet, dass** für das Blockieren der Verschiebbarkeit des Einstellelements (4) längs des Schiebeteiles (2) eine Zahneinrichtung (14 oder 15) an dem Wippenelement (6; 7) oder an dem Schiebeteil (2) vorgesehen ist und dass diese Zahneinrichtung (14 oder 15) in der die Verschiebbarkeit des Einstellelements (4) blockierenden zweiten Drehposition des Einstellelements (4) an dem ihr gegenüber liegenden Bereich des Schiebeteiles (2) bzw. des Wippenelements (6; 7) anliegt oder in diesen Bereich eindringt.

3. Vorrichtung nach Anspruch 1, **dadurch gekennzeichnet, dass** für das Blockieren der Verschiebbarkeit des Einstellelements (4) längs des Schiebeteiles (2) sowohl an dem Wippenelement (6; 7) als auch an dem Schiebeteil (2) zueinander passende Zahneinrichtungen (14, 15) vorgesehen sind, die lediglich bei Eingriff ineinander in der genannten zweiten Drehposition des Einstellelements (4) die Verschiebbarkeit des Einstellelements (4) blockieren.

4. Vorrichtung nach Anspruch 2, **dadurch gekennzeichnet, dass** das Einstellelement (4) durch einen Einstellring (4) gebildet ist, der in seinem das Wippenelement (6; 7) aufnehmenden Innenbereich derart oval- bzw. sichelförmig geformt ist, dass die an dem Wippenelement (6; 7) oder an dem Schiebeteil (2) vorgesehene Zahneinrichtung (14 oder 15) lediglich in der genannten zweiten Drehposition an dem ihr gegenüber liegenden Bereich des Schiebeteiles (2) bzw. des Wippenelements (6; 7) anliegt oder in diesen Bereich eindringt.

5. Vorrichtung nach Anspruch 3, **dadurch gekennzeichnet, dass** das Einstellelement (4) durch einen Einstellring (4) gebildet ist, der in seinem das Wippenelement (6; 7) aufnehmenden Innenbereich derart oval- oder sichelförmig geformt ist, dass die an dem Wippenelement (6; 7) und dem Schiebeteil (2) zueinander passenden Zahneinrichtungen (14, 15) lediglich in der genannten zweiten Drehposition miteinander in Eingriff gebracht sind und die Verschiebbarkeit des Einstellelements (4) blockieren.

6. Vorrichtung nach Anspruch 4 oder 5, **dadurch gekennzeichnet, dass** der Einstellring (4) auf seiner Innenseite wenigstens einen abstehenden Rippenteil (16) aufweist, der durch Anlage an dem Wippenelement (6; 7) den Drehwinkel des Einstellringes (4) auf dem Schiebeteil (2) zu begrenzen gestattet.

7. Vorrichtung nach Anspruch 6, **dadurch gekennzeichnet, dass** der Drehwinkel bei zwei zumindest angenähert einander gegenüberliegenden Rippenteilen (16) einen Wert von etwa 180° abzüglich der Winkelbreite aufweist, über die sich das jeweilige Wippenelement (6; 7) erstreckt.

8. Vorrichtung nach einem der Ansprüche 4 bis 7, **dadurch gekennzeichnet, dass** der Einstellring (4) auf seiner Außenseite zumindest einen, vor-zugsweise drei Betätigungsnocken (17) trägt.

9. Vorrichtung nach Anspruch 8, **dadurch gekennzeichnet, dass** der jeweilige Betätigungsnocken (17) von dem Einstellring (4) radial absteht.

10. Vorrichtung nach einem der Ansprüche 1 bis 9, **dadurch gekenn**- **zeichnet**, dass das jeweilige Wippenelement (6, 7) in Längsrichtung des Schiebeteiles (2) verdrehungssicher geführt ist.

11. Medizinisches Handstück (20) für ein medizinisches Gerät, unter Verwendung einer ersten und einer zweiten Vorrichtung nach einem der vorhergehenden Ansprüche, wobei die erste Vorrichtung die Eindringtiefe eines ersten rohrförmigen Schiebeteiles (30; 21, 22) in einem ersten Aufnahmeteil (33) festzulegen gestattet und wobei die zweite Vorrichtung die Eindringtiefe eines zweiten rohrförmigen Schiebeteiles (40; 21', 22') in einem zweiten Aufnahmeteil (21) festzulegen gestattet, umfassend folgende Merkmale:
• die erste Vorrichtung und die zweite Vorrichtung sind derart hintereinander angeordnet, dass das erste Schiebeteil (30; 21, 22) der ersten Vorrichtung zumindest zum Teil als zweites Aufnahmeteil (21) für das zweite Schiebeteil (40; 21', 22') der zweiten Vorrichtung ausgebildet ist;
• das erste Schiebeteil (30; 21, 22) und das zweite Schiebeteil (40; 21', 22') sind jeweils von einem Einstellelement (24; 24') umgeben, relativ zu dem das jeweils zugehörige Schiebeteil (30; 21, 22; 40; 21', 22') verschiebbar ist;
• das erste Einstellelement (24) und das zweite Einstellelement (24') sind in ihrer jeweiligen Stellung mittels einer eigenen Klemmeinrichtung unter Festlegung der Eindringtiefe des zugehörigen ersten bzw. zweiten Schiebeteiles (30; 21, 22; 40; 21', 22') in dem dieses aufnehmenden ersten bzw. zweiten Aufnahmeteil (33; 21) blockierbar;
• jede Klemmeinrichtung ist dadurch gebildet, dass zwischen dem jeweiligen Einstellelement (24; 24') und dessen zugehörigen Schiebeteil (30; 21, 22; 40; 21', 22') zumindest ein Wippenelement (26, 27; 26', 27') angeordnet ist, welches durch Verdrehen des betreffenden Einstellelements (24, 24') quer zur Längsrichtung des zugehörigen Schiebeteiles (30; 21, 22; 40; 21', 22') zwischen einer die Verschiebbarkeit des Schiebeteiles (30; 21, 22; 40; 21', 22') in Längsrichtung relativ zu dem zugehörigen Einstellelement (24; 24') zulassenden ersten Drehposition und einer eine solche Verschiebbarkeit blockierenden zweiten Drehposition bewegbar ist, die von der ersten Drehposition verschieden ist;
• mit dem ersten Aufnahmeteil (33) und dem zweiten Schiebeteil (40; 21', 22') sind Elemente (50, 51) des medizinischen Gerätes verbunden.

12. Handstück nach Anspruch 11, **dadurch gekennzeichnet, dass** das medizinische Gerät dadurch gebildet ist, dass mit dem ersten Aufnahmeteil (33) eine medizinische Hohl- oder Aspirationsnadel (51) verbunden ist, die sich in Längsrichtung durch das erste Aufnahmeteil (33), das erste Schiebeteil (30; 21, 22) mit dem daran ausgebildeten zweiten Aufnahmeteil (21) und das zweite Schiebeteil (40; 21', 22') hindurch erstreckt, und dass mit dem zweiten Schiebeteil (40; 21', 22') eine die medizinische Hohl- oder Aspirationsnadel (51) umgebende Umhüllung (50) verbunden ist.

13. Handstück nach Anspruch 12, **dadurch gekennzeichnet, dass** am proximalen Endbereich des ersten Schiebeteiles (30; 21, 22) ein Abschluss-Verbindungselement, insbesondere ein Schraubverbindungselement (52) zur Verbindung mit der medizinischen Hohl- oder Aspirationsnadel (51) angeordnet ist.

14. Handstück nach einem der Ansprüche 11 bis 13, **dadurch gekenn**- **zeichnet**, dass das erste Schiebeteil (30; 21, 22) und das zweite Schiebeteil (40; 21', 22') jeweils eine oder mehrere Markierungen oder Skaleneinteilungen enthalten.

15. Handstück nach einem der Ansprüche 11 bis 14, **dadurch gekennzeichnet, dass** am distalen Endbereich des zweiten Schiebeteiles (40; 21', 22') ein Verbindungselement (44), insbesondere ein Schraubverbindungselement zum Anbringen an einem Gegen-Verbindungselement eines weiteren medizinischen Gerätes, insbesondere eines Arbeitskanals eines Endoskops vorgesehen ist.

16. Handstück nach einem der Ansprüche 11 bis 15, **dadurch gekenn**- **zeichnet**, dass am proximalen Endbereich des ersten Aufnahmeteiles (33) ein Anschluss-Verbindungselement (52), insbesondere ein Schraubverbindungselement, gegebenenfalls zum Anbringen eines weiteren medizinischen Gerätes angeordnet ist.

17. Handstück nach einem der Ansprüche 11 bis 16, **dadurch gekenn**- **zeichnet**, dass das erste Aufnahmeteil (33) als Griffteil (33) gestaltet ist.

18. Handstück nach einem der Ansprüche 11 bis 17, **dadurch gekennzeichnet, dass** der als zweites Aufnahmeteil (21) für das zweite Schiebeteil (40; 21', 22') der zweiten Vorrichtung ausgebildete Teil des ersten Schiebeteiles (30; 21, 22) als Griffteil (21) gestaltet ist.

## Claims

1. Device (1) for establishing the penetration depth of a tubular or rod-shaped sliding part (2), particularly of a carrier part of a medical aspiration needle, in a receiving part (3), by means of an adjustment element (4) which surrounds the sliding part (2), is adjustable in the longitudinal direction of the sliding part (2) and can be blocked in its respective position by means of a clamping mechanism (5), establishing the penetration depth of the sliding part (2) in the receiving part (3),
**characterized in that** the clamping mechanism (5) is formed by at least one rocker element (6; 7) being arranged between the adjustment element (4) and the sliding part (2), which rocker element (6; 7) is movable, by rotation of the adjustment element (4) transverse to the longitudinal direction of the sliding part (2), between a first rotational position, which permits the displaceability of the adjustment element (4) in the longitudinal direction of the sliding part (2), and a second rotational position, which blocks such displaceability and which is different from the first rotational position.

2. Device according to Claim 1, **characterized in that**, in order to block the displaceability of the adjustment element (4) along the sliding part (2), a toothed arrangement (14 or 15) is provided on the rocker element (6; 7) or on the sliding part (2), and **in that**, in the second rotational position of the adjustment element (4) blocking the displaceability of the adjustment element (4), this toothed arrangement (14 or 15) bears on the region of the sliding part (2) or of the rocker element (6; 7) lying opposite it, or penetrates into this region.

3. Device according to Claim 1, **characterized in that**, in order to block the displaceability of the adjustment element (4) along the sliding part (2), toothed arrangements (14, 15) that match each other are provided both on the rocker element (6; 7) and also on the sliding part (2), which toothed arrangements (14, 15) block the displaceability of the adjustment element (4) only when they engage in each other in said second rotational position of the adjustment element (4).

4. Device according to Claim 2, **characterized in that** the adjustment element (4) is formed by an adjustment ring (4) which, in its inner region receiving the rocker element (6; 7), is configured in an oval shape or crescent shape, in such a way that the toothed arrangement (14 or 15) provided on the rocker element (6; 7) or on the sliding part (2) bears on the region of the sliding part (2) or of the rocker element (6; 7) lying opposite it, or penetrates into this region, only in said second rotational position.

5. Device according to Claim 3, **characterized in that** the adjustment element (4) is formed by an adjustment ring (4) which, in its inner region receiving the rocker element (6; 7), is configured in an oval shape or crescent shape, in such a way that the toothed arrangements (14, 15) matching each other on the rocker element (6; 7) and on the sliding part (2) are brought into engagement with each other, and block the displaceability of the adjustment element (4), only in said second rotational position.

6. Device according to Claim 4 or 5, **characterized in that** the adjustment ring (4) has, on its inside, at least one projecting rib part (16) which, by bearing on the rocker element (6; 7), makes it possible to limit the angle of rotation of the adjustment ring (4) on the sliding part (2).

7. Device according to Claim 6, **characterized in that**, in the case of two rib parts (16) lying at least approximately opposite each other, the angle of rotation has a value of about 180° minus the angle width over which the respective rocker element (6; 7) extends.

8. Device according to one of Claims 4 to 7, **characterized in that** the adjustment ring (4) carries at least one actuation cam (17), preferably three actuation cams (17), on its outside.

9. Device according to Claim 8, **characterized in that** the respective actuation cam (17) projects radially from the adjustment ring (4).

10. Device according to one of Claims 1 to 9, **characterized in that** the respective rocker element (6, 7) is guided in a manner secure against twisting in the longitudinal direction of the sliding part (2).

11. Medical handpiece (20) for a medical appliance, using a first and a second device according to one of the preceding claims, wherein the first device makes it possible to establish the penetration depth of a first tubular sliding part (30; 21, 22) in a first receiving part (33), and wherein the second device makes it possible to establish the penetration depth of a second tubular sliding part (40; 21', 22') in a second receiving part (21), comprising the following features:
• the first device and the second device are arranged one behind the other, in such a way that the first sliding part (30; 21, 22) of the first device is designed at least in part as a second receiving part (21) for the second sliding part (40; 21' 22') of the second device;
• the first sliding part (30; 21, 22) and the second sliding part (40; 21' 22') are each surrounded by an adjustment element (24; 24'), relative to which the respectively associated sliding part (30; 21, 22; 40; 21', 22') is displaceable;
• the first adjustment element (24) and the second adjustment element (24') can be blocked in their respective position by means of a dedicated clamping mechanism, establishing the penetration depth of the associated first or second sliding part (30; 21, 22; 40; 21', 22') in the first or second receiving part (33; 21), respectively, that receives it;
• each clamping mechanism is formed by at least one rocker element (26, 27; 26', 27') being arranged between the respective adjustment element (24; 24') and its associated sliding part (30; 21, 22; 40; 21', 22'), which rocker element (26, 27; 26', 27') is movable, by rotation of the relevant adjustment element (24, 24') transverse to the longitudinal direction of the associated sliding part (30; 21, 22; 40; 21' 22'), between a first rotational position, which permits the displaceability of the sliding part (30; 21, 22; 40; 21', 22') in the longitudinal direction relative to the associated adjustment element (24; 24'), and a second rotational position, which blocks such displaceability and which is different from the first rotational position;
• elements (50, 51) of the medical appliance are connected to the first receiving part (33) and to the second sliding part (40; 21', 22').

12. Handpiece according to Claim 11, **characterized in that** the medical appliance is formed by the fact that a medical hollow needle or aspiration needle (51) is connected to the first receiving part (33) and extends in the longitudinal direction through the first receiving part (33), the first sliding part (30; 21, 22), with the second receiving part (21) formed thereon, and the second sliding part (40; 21', 22'), and by the fact that a sheath (50) that surrounds the medical hollow needle or aspiration needle (51) is connected to the second sliding part (40; 21', 22').

13. Handpiece according to Claim 12, **characterized in that** an end connection element, particularly a screw connection element (52) for connection to the medical hollow needle or aspiration needle (51), is arranged at the proximal end region of the first sliding part (30; 21, 22).

14. Handpiece according to one of Claims 11 to 13, **characterized in that** the first sliding part (30; 21, 22) and the second sliding part (40; 21', 22') each contain one or more markings or scale divisions.

15. Handpiece according to one of Claims 11 to 14, **characterized in that** a connection element (44), particularly a screw connection element, for mounting on a mating connection element of another medical appliance, particularly a work channel of an endoscope, is provided at the distal end region of the second sliding part (40; 21', 22').

16. Handpiece according to one of Claims 11 to 15, **characterized in that** a connection element (52), particularly a screw connection element, is arranged at the proximal end region of the first receiving part (33), if appropriate for attaching a further medical appliance.

17. Handpiece according to one of Claims 11 to 16, **characterized in that** the first receiving part (33) is configured as a grip part (33).

18. Handpiece according to one of Claims 11 to 17, **characterized in that** that part of the first sliding part (30; 21, 22) designed as a second receiving part (21) for the second sliding part (40; 21', 22') of the second device is configured as a grip part (21).

## Revendications

1. Dispositif (1) pour établir la profondeur de pénétration d'une pièce coulissante en forme de tube ou de tige (2), en particulier d'une pièce de support d'aiguille d'aspiration médicale, dans une pièce réceptrice (3) au moyen d'un élément d'ajustement (4) entourant la pièce coulissante (2), lequel élément d'ajustement peut être réglé dans la direction longitudinale de la pièce coulissante (2) et peut être bloqué dans sa position respective au moyen d'un dispositif de serrage (5) en fixant la profondeur de pénétration de la pièce coulissante (2) dans la pièce réceptrice (3),
**caractérisé en ce que** le dispositif de serrage (5) est réalisé de telle sorte qu'au moins un élément de bascule (6 ; 7) soit disposé entre l'élément d'ajustement (4) et la pièce coulissante (2), lequel élément de bascule, lors de la rotation de l'élément d'ajustement (4) transversalement à la direction longitudinale de la pièce coulissante (2), peut être déplacé entre une première position de rotation permettant le déplacement de l'élément d'ajustement (4) dans la direction longitudinale de la pièce coulissante (2) et une deuxième position de rotation bloquant un tel déplacement, laquelle est différente de la première position de rotation.

2. Dispositif selon la revendication 1, **caractérisé en ce que** pour le blocage du déplacement de l'élément d'ajustement (4) le long de la pièce coulissante (2), un dispositif denté (14 ou 15) est prévu sur l'élément de bascule (6 ; 7) ou sur la pièce coulissante (2) et **en ce que** ce dispositif denté (14 ou 15) s'applique dans la deuxième position de rotation de l'élément d'ajustement (4) bloquant le déplacement de l'élément d'ajustement (4) contre la région qui lui est opposée de la pièce coulissante (2) ou de l'élément de bascule (6 ; 7) ou pénètre dans cette région.

3. Dispositif selon la revendication 1, **caractérisé en ce que** pour le blocage du déplacement de l'élément d'ajustement (4) le long de la pièce coulissante (2) ainsi que contre l'élément de bascule (6 ; 7) et également contre la pièce coulissante (2), des dispositifs dentés (14, 15) adaptés l'un à l'autre sont prévus, lesquels bloquent le déplacement de l'élément d'ajustement (4) uniquement lors de l'engagement l'un dans l'autre dans ladite deuxième position de rotation de l'élément d'ajustement (4).

4. Dispositif selon la revendication 2, **caractérisé en ce que** l'élément d'ajustement (4) est formé par une bague d'ajustement (4) dont la région intérieure recevant l'élément de bascule (6 ; 7) est formée sous forme ovale ou en forme de croissant de telle sorte que le dispositif denté (14 ou 15) prévu sur l'élément de bascule (6 ; 7) ou sur la pièce coulissante (2) s'applique seulement dans ladite deuxième position de rotation contre la région qui lui est opposée de la pièce coulissante (2) ou de l'élément de bascule (6 ; 7) ou pénètre dans cette région.

5. Dispositif selon la revendication 3, **caractérisé en ce que** l'élément d'ajustement (4) est formé par une bague d'ajustement (4) dont la région intérieure recevant l'élément de bascule (6 ; 7) est formée sous forme ovale ou en forme de croissant de telle sorte que les dispositifs dentés (14, 15) adaptés l'un à l'autre sur l'élément de bascule (6 ; 7) et la pièce coulissante (2) soient amenés en prise l'un avec l'autre uniquement dans ladite deuxième position de rotation et bloquent le déplacement de l'élément d'ajustement (4).

6. Dispositif selon la revendication 4 ou 5, **caractérisé en ce que** la bague d'ajustement (4) présente sur son côté intérieur au moins une partie d'arête saillante (16) qui permet de limiter l'angle de rotation de la bague d'ajustement (4) sur la pièce coulissante (2) par application contre l'élément de bascule (6 ; 7).

7. Dispositif selon la revendication 6, **caractérisé en ce que** l'angle de rotation, lorsque les deux parties d'arête sont au moins approximativement en regard l'une de l'autre (16), présente une valeur d'environ 180° moins la largeur de l'angle sur laquelle s'étend l'élément de bascule respectif (6 ; 7).

8. Dispositif selon l'une quelconque des revendications 4 à 7, **caractérisé en ce que** la bague d'ajustement (4) porte sur son côté extérieur au moins une, de préférence trois, cames d'actionnement (17).

9. Dispositif selon la revendication 8, **caractérisé en ce que** la came d'actionnement respective (17) fait saillie radialement depuis la bague d'ajustement (4).

10. Dispositif selon l'une quelconque des revendications 1 à 9, **caractérisé en ce que** l'élément de bascule respectif (6, 7) est guidé de manière fixée en rotation dans la direction longitudinale de la pièce coulissante (2).

11. Pièce à main médicale (20) pour un appareil médical, utilisant un premier et un deuxième dispositif selon l'une quelconque des revendications précédentes, dans laquelle le premier dispositif permet d'établir la profondeur de pénétration d'une première pièce coulissante tubulaire (30 ; 21, 22) dans une première pièce réceptrice (33) et dans laquelle le deuxième dispositif permet d'établir la profondeur de pénétration d'une deuxième pièce coulissante tubulaire (40 ; 21', 22') dans une deuxième pièce réceptrice (21), comprenant les caractéristiques suivantes :
- le premier dispositif et le deuxième dispositif sont disposés l'un derrière l'autre de telle sorte que la première pièce coulissante (30 ; 21, 22) du premier dispositif soit réalisée au moins en partie en tant que deuxième partie réceptrice (21) pour la deuxième pièce coulissante (40 ; 21', 22') du deuxième dispositif ;
- la première pièce coulissante (30 ; 21, 22) et la deuxième pièce coulissante (40 ; 21', 22') sont à chaque fois entourées par un élément d'ajustement (24 ; 24') par rapport auquel la pièce coulissante associée respective (30 ; 21, 22 ; 40 ; 21', 22') peut coulisser ;
- le premier élément d'ajustement (24) et le deuxième élément d'ajustement (24') peuvent être bloqués dans leur position respective au moyen d'un dispositif de serrage propre en fixant de la profondeur de pénétration de la première ou de la deuxième pièce coulissante associée (30 ; 21, 22 ; 40 ; 21', 22') dans la première ou la deuxième partie réceptrice (33 ; 21) recevant celle-ci ;
- chaque dispositif de serrage est formé en ce qu'entre l'élément d'ajustement respectif (24 ; 24') et sa pièce coulissante associée (30 ; 21, 22 ; 40 ; 21', 22') est disposé au moins un élément de bascule (26, 27 ; 26', 27'), lequel peut être déplacé par rotation de l'élément d'ajustement concerné (24, 24') transversalement à la direction longitudinale de la pièce coulissante associée (30 ; 21, 22 ; 40 ; 21', 22') entre une première position de rotation permettant le déplacement de la pièce coulissante (30 ; 21, 22 ; 40 ; 21', 22') dans la direction longitudinale par rapport à l'élément d'ajustement associé (24 ; 24') et une deuxième position de rotation bloquant un tel déplacement, laquelle est différente de la première position de rotation ;
- des éléments (50, 51) de l'appareil médical sont connectés à la première pièce réceptrice (33) et à la deuxième pièce coulissante (40 ; 21', 22').

12. Pièce à main selon la revendication 11, **caractérisée en ce que** l'appareil médical est formé **en ce qu'**une aiguille creuse ou d'aspiration médicale (51) est connectée à la première pièce réceptrice (33), laquelle aiguille s'étend dans la direction longitudinale à travers la première pièce réceptrice (33), la première pièce coulissante (30 ; 21, 22) avec la deuxième pièce réceptrice (21) réalisée sur celle-ci et la deuxième pièce coulissante (40 ; 21', 22'), et **en ce qu'**une gaine (50) entourant l'aiguille creuse ou d'aspiration médicale (51) est connectée à la deuxième pièce coulissante (40 ; 21', 22').

13. Pièce à main selon la revendication 12, **caractérisée en ce qu'**un élément de connexion de terminaison, en particulier un élément de connexion par vissage (52), est disposé au niveau de la région d'extrémité proximale de la première pièce coulissante (30 ; 21, 22) pour la connexion à l'aiguille creuse ou d'aspiration médicale (51).

14. Pièce à main selon l'une quelconque des revendications 11 à 13, **caractérisée en ce que** la première pièce coulissante (30 ; 21, 22) et la deuxième pièce coulissante (40 ; 21', 22') contiennent chacune un ou plusieurs marquages ou une ou plusieurs graduations d'échelle.

15. Pièce à main selon l'une quelconque des revendications 11 à 14, **caractérisée en ce qu'**un élément de connexion (44), en particulier un élément de connexion par vissage, est prévu au niveau de la région d'extrémité distale de la deuxième pièce coulissante (40 ; 21', 22') en vue du montage sur un élément de connexion conjugué d'un autre appareil médical, en particulier d'un canal de travail d'un endoscope.

16. Pièce à main selon l'une quelconque des revendications 11 à 15, **caractérisée en ce qu'**un élément de connexion de raccordement (52), en particulier un élément de connexion par vissage, est disposé au niveau de la région d'extrémité proximale de la première pièce réceptrice (33) éventuellement pour le montage d'un autre appareil médical.

17. Pièce à main selon l'une quelconque des revendications 11 à 16, **caractérisée en ce que** la première pièce réceptrice (33) est réalisée sous forme de pièce de préhension (33).

18. Pièce à main selon l'une quelconque des revendications 11 à 17, **caractérisée en ce que** la pièce de la première pièce coulissante (30 ; 21, 22) réalisée en tant que deuxième pièce réceptrice (21) pour la deuxième pièce coulissante (40 ; 21', 22') du deuxième dispositif est réalisée sous forme de pièce de préhension (21).
